(19) Europäisches Patentamt
European Patent Office
Office européen des brevets

(11) Publication number: **0 233 441 A1**

# (12) EUROPEAN PATENT APPLICATION

(21) Application number: **86850439.0**

(22) Date of filing: **15.12.86**

(51) Int. Cl.[4]: **C07H 7/02**, C07C 59/105, C07C 59/125, C07C 69/675, C07C 69/708, A61K 31/70

The title of the invention has been amended (Guidelines for Examination in the EPO, A-III, 7.3).

(30) Priority: **20.12.85 SE 8506095**

(43) Date of publication of application: **26.08.87 Bulletin 87/35**

(84) Designated Contracting States: **ES GR**

(71) Applicant: **Astra Läkemedel Aktiebolag**
**Strängnäsvägen 44**
**S-151 85 Södertälje(SE)**

(72) Inventor: **Bondesson, Ulf Göran**
**Brinkstigen 6**
**S-141 70 Huddinge(SE)**
Inventor: **Claesson, Alf**
**Lilldalsvägen 17A**
**S-144 00 Rönninge(SE)**
Inventor: **Gustafsson, Kent Gustaf Sigvard**
**Harvarvägen 21**
**S-150 23 Enhörna(SE)**
Inventor: **Luthman, Ingrid Kristina**
**428 N. Street 6**
**Bethlehem, PA 18018(US)**

(74) Representative: **Hjertman, Ivan T. et al**
**AB ASTRA Patent and Trade Mark Department**
**S-151 85 Södertälje(SE)**

## (54) New enzyme inhibitor.

(57) A compound of the formula

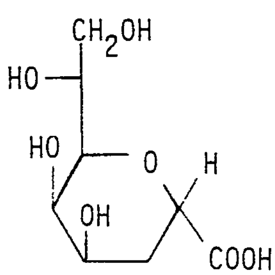

I

or physiologically acceptable salts thereof, processes for its preparation, chemical intermediates and their preparation, and pharmaceutical preparations containing it. The compound exerts an inhibitory activity on the enzyme CMP-KDO synthetase.

EP 0 233 441 A1

## New Enzyme Inhibitor

The present invention relates to a new anhydrooctonic acid, and salts thereof, which is a powerful inhibitor of bacterial lipopolysaccharide synthesis. The compound has potential use as an antibacterial agent and as an intermediate or starting material in the preparation of antibacterial agents. The invention also relates to methods for the preparation of the anhydrooctonic acid as well as to new intermediates useful for the preparation of anhydrooctulosonic acids. The invention further relates to preparation of pharmaceutical preparations containing the said acid or salts thereof.

### Field of the invention

### Background of the invention

Lipopolysaccharides (LPS) form a unique class of macromolecules which are characteristic of Gram-negative bacteria. They constitute the outer part of the outer membrane of the microorganism and are involved in the interaction of the cell with the environment. LPS thus plays a critical role in determining the antigenicity, toxicity, adhesiveness, invasiveness, and penetrability of the cell. The outer membrane constitutes an important structure of the Gram-negative bacterial cell by protecting it against the environment, e.g. the host defense system and antibacterial agents.

The structure of LPS in Salmonella typhimurium is shown schematically in the Figure below.

(Tetrasaccharide)$_8$-Hex-Hex-Hep-Hep-KDO-KDO-Lipid A

O-Antigen Core

The Lipid A constitutes the innermost part of LPS. Hex = Hexose, Hep = Heptose, KDO = 3-deoxy-D-manno-octulosonic acid.

Due to the importance of LPS for bacterial survival it might be expected that damage to it would affect the viability of the microbial cell. It has thus been found that mutants defective in 3-deoxy-D-manno-octulosonic acid (KDO) biosynthesis are no longer viable. Enzymes involved in the biosynthesis of this acid might therefore constitute valuable targets for chemical compounds, which by inhibition of the enzymes involved would act as novel antimicrobial agents.

### Prior art

3-Deoxy-D-manno-2-octulusonic acid (KDO) is described in Advances in Carbohydrate Chemistry and Biochemistry, 38, 323-388 (1981).

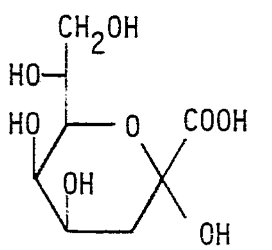

KDO

Some analogues of KDO are reported to have an inhibiting effect upon the enzyme CMP-KDO synthetase (p. 388).

Ray et al (ACS Symposium Series 231, 141-169) describes the synthesis of analogues of 3-deoxy-D-manno-2-octulosonate (KDO) and their use in inhibiting the biosynthesis of the LPS of Escherichia coli. Analogues of KDO have been synthesized and tested as inhibitors or alternative substrates for CMP-KDO synthetase. The substrate analogues were weak inhibitors of the enzymes involved in KDO biosynthesis.

In J. Med. Chem., 27, 717-26 (1984) compounds are described which are reported to inhibit arabinose-5-phosphate isomerase in the biosynthesis of lipopolysaccharide.

## Disclosure of the invention

The present invention relates to a new compound, 2,6-anhydro-3-deoxy-D-glycero-D-talo-octonic acid with the formula I and physiologically acceptable salts thereof.

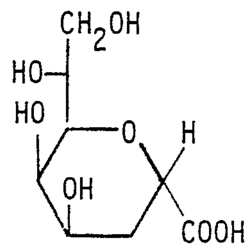

I

It has surprisingly been found that the compound of the invention exhibits a strong inhibitory activity on the enzyme cytidine monophosphate-3-deoxy-D-manno-octulosonate synthetase (CMP-KDO synthetase). This activity suggests a potential value of the compound as an antimicrobially active agent. The invention takes into consideration that compounds which structurally deviate from the formula I, after administration to a living organism, may be transported into the bacteria and there be transformed to the compound of formula I by hydrolysis or other chemical reactions, and in this structural form exhibit its inhibitory activity on the CMP-KDO synthetase. The compound of the invention is thus useful as an intermediate in the preparation of such compounds in order to obtain an antimicrobially or antibacterially active agent.

The compound of the invention is preferably used as a salt, e.g. a metal salt or a salt with an organic base,preferably the ammonium salt of the formula

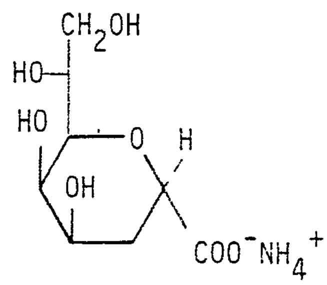

## Methods of preparation

The compound of the invention may be obtained by one of the following methods constituting a further aspect of the invention.

A. Epimerization (base-catalyzed isomerization) of a compound of the formula

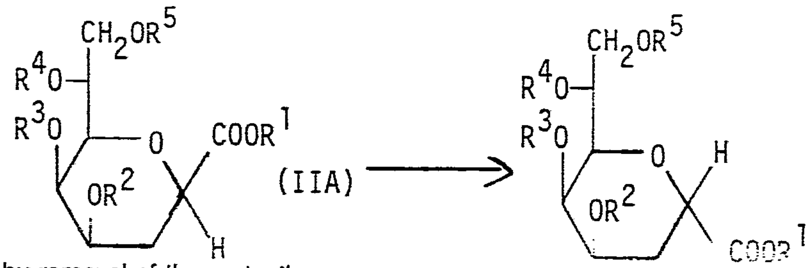

(IIA) (IIB)

followed by removal of the protecting groups wherein $R^2$-$R^5$ are the same or different and selected from hydrogen or among the protective groups which are stable towards base, such as arylalkyl, or cyclic acetal preferably benzyl or acetonide, and $R^1$ is an alkyl group. The epimerization may be performed in an alcoholic solvent, e.g. methanol or ethanol containing a metal alkoxide corresponding to R'O, or by treatment with a metal amide such as lithium diisopropylamide in a suitable solvent such as tetrahydrofuran or diethyl ether.

B. Cyclization of a partly protected compound of the formula

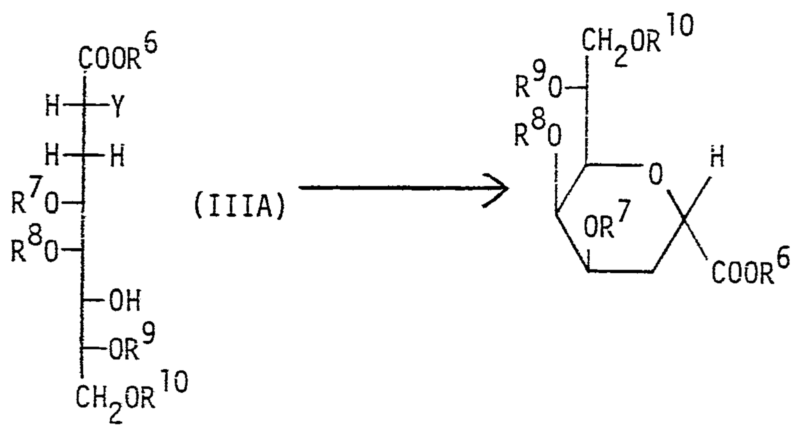

(IIIA) (IIIB)

wherein Y is a leaving group, e.g. a halogen atom such as chloro, bromo or iodo, an aryl-or alkylsulfonyloxy group, or a phosphoniooxy group $(R^{11})_3\overset{+}{P}O$-where $R^{11}$ is phenyl or alkyl containing I-4 carbon atoms. $R^7$-$R^{10}$ are hydrogen, acyl, arylalkyl such as benzyl, 2-tetrahydropyranyl, cyclic acetal such as acetonide or a protecting group known in the art. $R^6$ is hydrogen, an alkyl group containing I-6 carbon atoms, arylalkyl or a protecting group known in the art. A great variety of protecting groups are known to those skilled in the art by for instance "Protecting Groups in Organic Chemistry" T.W. Greene, Wiley I98I. The cyclization is initiated by adding a base such as potassium carbonate or metal alkoxide to a solution of the compound of formula IIIA in an alcoholic solvent, e.g. methanol or ethanol,or a mixture including the said alcohols at room temperature to 5O°C,or by treatment of the compound IIIA with sodium hydride in an inert solvent such as tetrahydrofuran or toluene.

The protecting groups of the claimed acid may be removed by one or more chemical reactions known to those skilled in the art,e.g. hydrolysis, to obtain the compound of the formula I.

C. Reduction of a KDO derivative of the formulas IVA or IVB, followed by removal of protecting groups

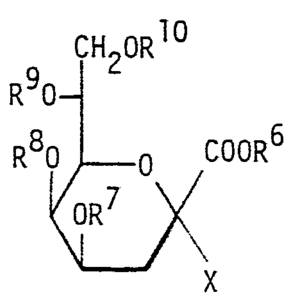

IVA

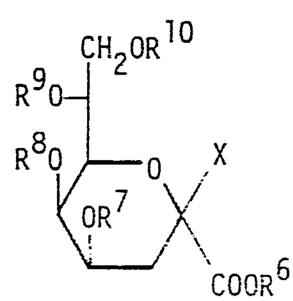

IVB

wherein X is a halogen atom, preferably chloro or bromo, or a sulfur-containing group such as alkylthio, arylthio or aryl-SCOO or thiocarbonyl, $R^6$-$R^{10}$ are as defined under B.

The reduction is preferably carried out using hydrogen in combination with a metal catalyst such as palladium, platina or nickel. Alternatively, the reduction can be carried out under radical-generating conditions involving a hydride reducing agent such as trialkyltin hydride.

The reduction with hydrogen may be performed in a suitable solvent, such as ethyl acetate, toluene, acetone or dimethyl formamide (DMF) at a temperature between O°C and 5O°C in the presence of a tertiary amine such as pyridine or triethylamine.

## Salts

Physiologically acceptable salts of compounds of the invention are prepared by methods known in the art. The salts are novel compounds and comprise a further aspect of the invention. Addition salts can be prepared by treating the compound of the invention with, for example, ammonia. Metal salts can be prepared by treating a metal hydroxide with a compound of the invention. Examples of metal salts which can be prepared in this way are salts containing Li, Na and K. A less soluble metal salt can be precipitated from a solution of a more soluble salt by addition of a suitable metal compound.

## Pharmaceutical preparations

Pharmaceutical preparations of the compound of the invention constitute a further aspect of the invention.

The compound of the invention may be administered in a form aimed at facilitating the transport of the compound of the invention across the bacterial cell wall.

The compounds of the invention can be administered in the form of a pharmaceutical preparation comprising the active ingredient in the form of the original compound or optionally in the form of a pharmaceutically acceptable salt thereof in association with a pharmaceutically acceptable carrier.

## Intermediates

Some of the intermediates or starting materials used in the methods A-C above are known. However, certain intermediates or starting materials are novel and constitute a further aspect of the invention. Thus, in one aspect the invention is related to novel compounds of the formula

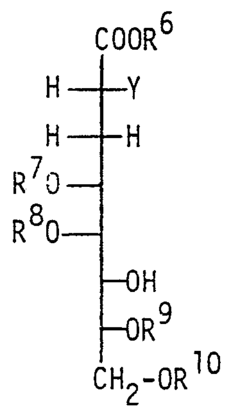

IIIA

wherein Y is a leaving group such as a halogen atom, a sulfonate group or a phosphoniooxy group and $R^6$-$R^{10}$ are same or different and selected from hydrogen or protecting groups as described above; and to the methods of preparing said chemical intermediates. The preparation of the intermediates will be illustrated by specific examples below.

## Methods of preparation of intermediates

The intermediate, of the formula

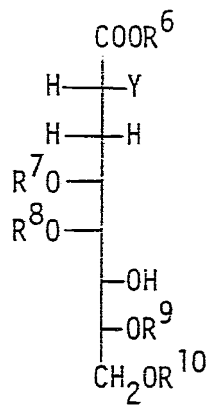

IIIA

wherein Y and $R^6$-$R^{10}$ are as stated above, may be obtained by one of the following methods.

D. Formation and halogenation of a metal enolate

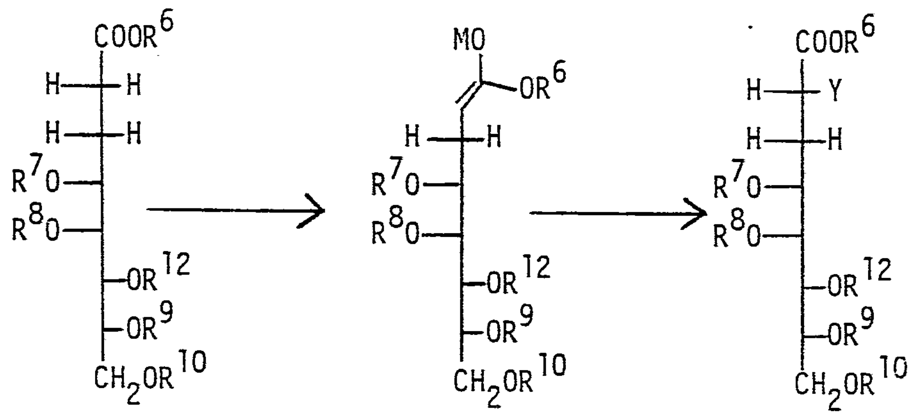

wherein $R^6$-$R^{10}$ are as stated in Method B, $R^{12}$ is a protecting group, which is stable towards base but is removable under conditions where $R^6$-$R^{10}$ are stable, and M is a metal cation. This method has been described by Rathke et al. Tetrah. Lett. 1971, 3995. $R^{12}$ is generally selected from the substituted silyl groups such as trimethylsilyl, substituted benzyl groups such as 2,4-dimethoxybenzyl or alkylthioalkyl groups such as methylthiomethyl. M is a metal or mono cation such as lithium or sodium or a metal having additional ligands such as zinc, tin, boron or titanium which confer suitable reactivity on the enolate.

The starting material in this synthesis can be prepared from a suitably protected D -manno-2-octenoate by catalytic hydrogenation and, for example, silylation according to standard procedures known to those skilled in the art. An example of a synthesis of such an octenoate is given by Collins et al. in J.C.S. Chem. Commn. 1981, 1139 who reported the preparation of ethyl 3,4;7,8-di-O-isopropylidene- D -manno-2-octenoate.

The enolate is formed and allowed to react in an inert organic solvent such as tetrahydrofuran, diethyl ether or tetramethylurea at a temperature of between -120-0°C.

Suitable reagents by which to form the enolate may be chosen among the metal amides, such as lithium diisopropylamide, or metal triflates, such as tin(II) triflate, in the presence of a tertiary amine. A suitable modifying metal salt such as zinc chloride may also be added to the solution of a lithium enolate. The addition of a modifying salt in a halogenation of an enolate is new and constitutes a further valuable aspect of the invention.

After reaction and work-up the product is separated from its C-2 epimer, for example by chromatography on silica gel.

The group $R^{12}$ may be removed by methods known to those skilled in the art and the claimed compound isolated thereafter. We have found, however, that it is more convenient to postpone this step until the intermediate will be cyclized and then instead to perform the deprotection and the cyclization as an one-pot synthesis.

E. Hydrosilylation of a D-manno-2-octenoate and reacting the formed silyl ketene acetal with a halogen or pseudohalogen.

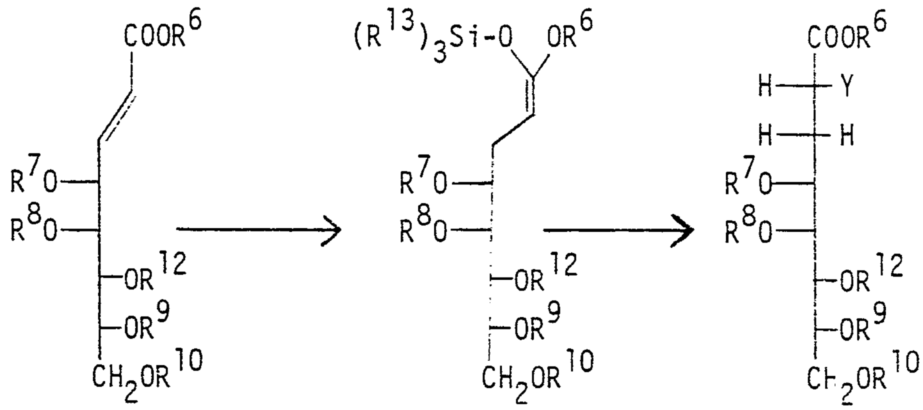

$R^6$-$R^{12}$ and Y are as described above and $R^{13}$ is a lower alkyl group such as ethyl.

The starting octenoate can be prepared by standard techniques, such as for example trimethylsilylation, from known compounds such as the one used as starting material in Method D.

The hydrosilylation is performed in excess trialkylsilane as the solvent or in an inert solvent such as toluene or tetrahydrofuran at a temperature of between 15-100°C using a trialkyl-or diarylsilane such as triethylsilane in the presence of a noble metal catalyst such as tris-triphenylphosphine rhodium chloride.

After reaction is complete pyridine or another suitable base is added and the mixture is treated with a halogen such as bromine or, preferably, with a pseudohalogen such as iodine chloride. The product is purified and treated as described above.

F. Hydrosilylation of an octenoate and reacting the formed silyl ketene acetal with a peracid

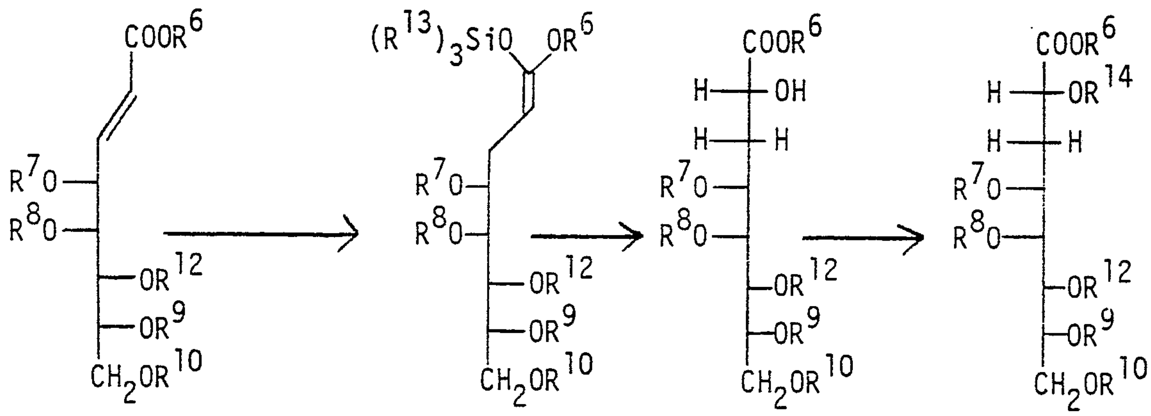

$R^6$-$R^{13}$ and Y are as described above and $R^{14}$ is an aryl-or alkylsulfonyloxy group as described above. The starting material can be obtained as described in Methods D and E. The hydrosilylation is carried out as described above. The silyl ketene acetal is then oxidized by using a peracid such as 3-chloroperbenzoic acid. After purification by chromatography the resulting silyl ether is treated with a fluoride salt in a suitable solvent such as methanol. The resulting alcohol is then converted into a sulfonate using methods known to those skilled in the art. $R^{12}$ is then removed as described above.

## Working examples

### Preparation of intermediates or starting material for use in Method B

Example I Ethyl 2,3-dideoxy-4,5;7,8-di-O-isopropylidene-6-O-(trimethylsilyl)- D -manno-octonate

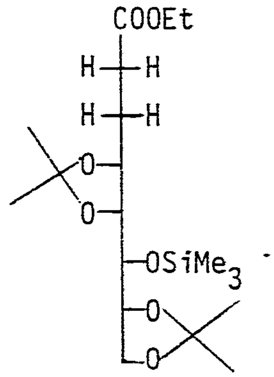

Ethyl 2,3-dideoxy-4,5;7,8-di-O-isopropylidene-D -manno-octenoate, which is a known compound - (Collins et al. Chem. Soc. Chem. Comm. 1981, 1139) is hydrogenated over 10% palladium-on-charcoal and then silylated using chlorotrimethylsilane in pyridine-ether to give after silica gel chromatography the title compound as a syrup in 90% yield. $[\alpha]_D$ +38.7 (c 1.04, $CHI_3$). $^{13}$C-NMR ($CHCl_3$, TMS-O in product as zero ppm): δ172.6, 108.6, 106.9, 76.2, 75.8, 74.9, 71.0, 66.2, 59.4, 29.9, 27.3, 25.3, 25.1, 24.5, 13.5.

Example 2 Ethyl 4,5;7,8-di-O-isopropylidene-2,3-dideoxy-2-iodo-6-O-(trimethylsilyl)-D -glycero-D -galacto-octonate

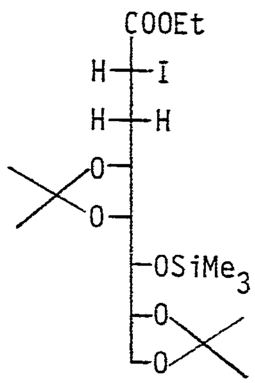

The compound from Example 1 (1.0 g;2.5 mmol) in 10 ml of tetrahydrofuran (THF) was added during 5 min to a stirred solution of lithium diisopropylamide (2.8 mmol) in 70 ml of THF, which was kept at -78°C under nitrogen. After 15 min dry $ZnCl_2$ (0.34 g; 2.5 mmol) was added in one portion and the mixture was stirred for 1 hour. While keeping the temperature of the reaction mixture at -70°C a solution of iodine (0.76 g; 3 mmol) in 10 ml of THF was added. The temperature was allowed to rise to about 0°C and solvents were evaporated under vacuum while keeping the temperature below 20°C. The residue was dissolved in 100 ml of ether -light petrol (1:4) and treated with 5 ml of a saturated solution of sodium thiosulfate and sodium hydrogen carbonate in water for 5 min. The mixture was dried over $Na_2SO_4$.

Chromatography on silica gel (light petrol -ether, 3:1) gave the title compound as a syrup and its C-2 epimer having talo configuration (0.60 and 0.38 g, respectively; 74% total yield). $R_f$ values in the same solvent system were 0.55 and 0.44, respectively.

Physical data: $[\alpha]_D$62° ( c1.10, $CHCl_3$). $^{13}$C-NMR: δ171.0, 109.6, 107.6, 80.2, 77.0, 76.3, 72.0, 67.7, 61.3, 35.7, 28.0, 26.2, 25,8, 25.1, 19.9 (CHI), 13.5 ppm.

Example 3 Ethyl 2,6-anhydro-2,3-dideoxy-2-iodo-4,5;7,8-diisopropylidene- D -glycero- D -talo-octonate

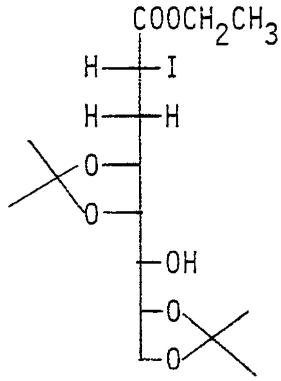

The galacto iodide from example 2 was allowed to react with 0.4 equivalents of tetrabutylammonium fluoride at room temperature in a 9:1 mixture of ethyl acetate and ethanol until all starting material had disappeared (1-5 min). This treatment removed the trimethylsilyl group to give the title compound in 90% yield after purification on silica gel (pentane ether, 1:1) $^{13}C$-NMR ($CHCl_3$): δ171.1, 109.3, 108.2, 76.5, 76.2, 76.2, 70.6, 67.3, 61.6, 35.4, 27.0, 26.7, 25.2, 24.8, 20.5, 13.6 IR: 3400 $cm^{-1}$ (strong).

Preparation of Partially Protected Final Products

Example 4 Methyl 4,5,7,8-tetra-O-acetyl-2,6-anhydro-3-deoxy-D -glycero-D -talo-octonate (Method C)

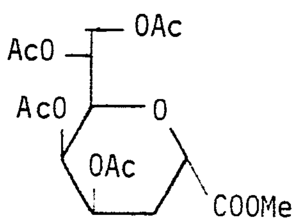

A mixture of 2.0 g (4.6 mmol) of methyl 4,5,7,8,tetra-O-acetyl-2-chloro-2,3-dideoxy-- D -manno-2-octulosonate (Bhattacharjee et al., Biochemistry 1978, 17, 645) in 25 ml of toluene containing 1 ml of pyridine and 1 g of 10% palladium-on-charcoal was hydrogenated in a Parr apparatus at 40 psi for 8 h. After filtration and evaporation of solvents the residue was chromatographed on silica gel with ether-pentane 2:1 as eluent to give 1.40 g (68%) of the title compound. M.p. 102-104°C. $[\alpha]_D$ + 108.8° (c 2.17, $CHCl_3$). $^{13}C$-NMR ($CHCl_3$); δ170.8-169.6 (C1 + acetyl), 72.3 (C2), 70.6 (C6), 68.1, 66.8, 65.0, 62.6 (C8), 52.5 (MeO), 26.5 (C3). Anal. Calc. for $C_{17}H_{24}O_{11}$ C, 50.50; H, 5.98. Found: C, 50.5; H, 6.2. From the reaction mixture was also isolated 135 mg (6%) of the C-2 epimer with galacto configuration which was briefly described by Unger et al. - (Communication at the 9th International Symposium on Carbohydrate Chemistry, London 1978. Abstract No B49).

Deprotection

Example 5 Methyl 2,6-anhydro-3-deoxy- D -glycero-D -talo-octonate

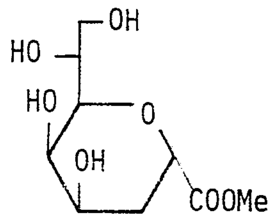

The tetraacetate-methyl ester from Example 4 was treated with 0.1 equivalents of sodium methoxide in dry methanol for 1 h at room temperature. Methanol-washed $H^+$ ion-exchange resin was added, the mixture was filtered and solvents were evaporated to give crystalline title compound (100%). M.p. 74-5°C; -$[\alpha]_D$ + 76° (c 2.40, MeOH). $^{13}$C-NMR ($D_2O$, dioxan ref. 67.4); δ174.4, 75.6, 73.4, 70.2, 67.2, 67.0, 64.3, 53.6, 28.4. +

Example 6 Ethyl 2,6-anhydro-3-deoxy-4,5;7,8-di-O-isopropylidene- D -glycero- D -talo-octonate (Method B).

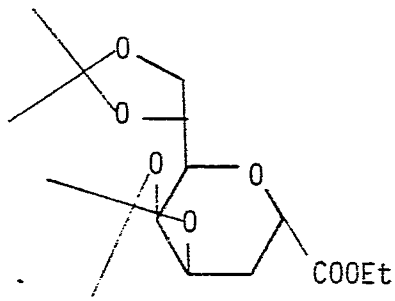

The compound from Example 3 was dissolved in a 9:1 mixture of ethyl acetate and ethanol and an excess of dry $K_2CO_3$ (about 3 equivalents) was then added and the stirred mixture was then left at room temperature for 30 h. Evaporation of solvents and silica gel chromatography (ether-pentane 1:1) gave a 95% yield of the title compound.

The D -talo-iodide from Example 2 was also converted in part to the title compound by first treating it in acetonitrile with a catalytic amount of tetrabutylammonium iodide to give an equilibrium mixture of the iodides, then separating the epimers and cyclizing the isolated galacto iodide as described in the present Example.

Deprotection

Example 7 Ethyl 2,6-anhydro-3-deoxy- D -glycero- D -talo -octonate.

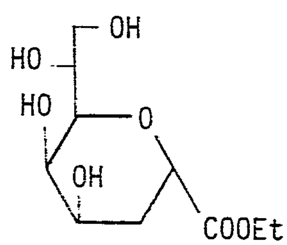

The bisacetonide (1.08 g) from example 6 was dissolved in 20 ml of methanol and 5 ml of trifluoroacetic acid were added. The mixture was left for 0.5 h and volatiles were then removed on a vacuum evaporator. The residue was dissolved in methanol which was removed under vacuum. This procedure was repeated three times. The residue crystallized to give 0.65 g of the title compound. M.p. 126-128°C. $^{13}$C-NMR (MeOH): δ173.4, 76.3, 73.8, 71.0, 68.0, 68.0, 65.6, 62.4, 29.6, 14.5.

Preparation of salts

Example 8 Ammonium 2,6-anhydro-3-deoxy- D -glycero - D -talo-octonate.

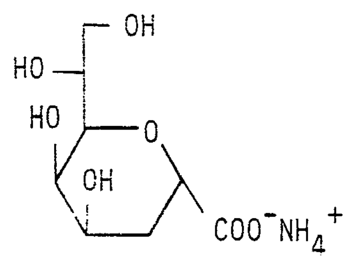

A solution of either of the compounds from Examples 5 or 7 in 2M NaOH was kept until all material was hydrolyzed. The solution was passed through an ammonium-saturated cation exchange resin followed by evaporation of water. The residue crystallized on standing in the refrigerator to give a monohydrate of the title compound (90%). M.p. 175-182° (decomposition). $[\alpha]_D$ + 69° ( c 1.68, $H_2O$). $^{13}C$-NMR ($D_2O$, t-BuOH ref$\delta$ = 30.6): 179.2, 75.0, 74.6, 70.1, 67.7, 67.3, 64.8, 29.4 ppm.

Anal. Calcd. for $C_8H_{19}NO_8$: C, 37.35; H, 7.44; N, 5.45. Found: C, 37.4; H, 7.28; N, 5.44.

## Biological Tests

The inhibitory effect of the compound of the invention on Gram-negative bacteria was tested using the method described below.

In the following the compound ammonium 2,6-anhydro-3-deoxy-D-glycero-D-talo-octonate is denoted compound I.

## Enzyme inhibition studies

Potential inhibitors were tested for inhibitory action against two key enzymes involved in the biosynthesis of bacterial LPS.

I. CMP-KDO synthetase (CKS) which catalyses the reaction

$$\text{KDO} + \text{CTP} \xrightarrow{\text{CKS}} \text{CMP-KDO} + \text{PPi}$$

CMP-KDO lipid A transferase (KLT) which transfers CMP-KDO to an intermediate in the biosynthesis of LPS known as lipid A precursor

$$\text{CMP-KDO} + \underset{\text{precursor}}{\text{lipid A}} \xrightarrow{\text{KLT}} \underset{\text{precursor}}{\text{KDO-lipid A}} + \text{CMP}$$

## CKS Assay

The CKS enzyme was prepared essentially as described by Osborne [J-.Biol.Chem. 242 (1978) 4904-4903] and the enzyme assayed by the method of Ghalambor and Heath (J.Biol.Chem. 241 (1966) 3216-3221).

## KLT Assay

The KLT enzyme was purified essentially by the method of Osborne (J.Biol.Chem. 252 (1977) 4895-4903). KLT activity was determined by monitoring the incorporation of [14-C]-labelled CMP-KDO into acid-insoluble product. The radio labelled CMP-KDO was generated in situ from CTP and [14-C]-KDO by the action of added CKS enzyme. The incubation mixture contained CKS enzyme, KLT enzyme, 110 mM Tris buffer pH 8.0, 10.6 mM NaCl, 0.5 mM CTP, 0.4%, Alfonic 1012-6 (Trade name), 0.16 mM [14-c] KDO and

lipid A precursor. The final reaction volume was 150 µl. The mixture was incubated at 25°C for 15 min., when 50 µl was transferred to a filter paper circle (Munktell No. 5,24 mm.) and then washed copiously with 10% TCA. The radioactivity adsorbed on the paper circle, which represents radiolabelled KDO bound to precursor was determined using liquid scintillation counting.

Results

Inhibition of CKS and KLT enzymes by compound I is given in Table I. The inhibitory action is expressed in percent of a control without inhibitor run simultaneously.

Table I Percentage of inhibition of CKS and KLT enzymes by compound I.

| Konc. Compound I (mM) | 10.16 | 2.54 | 0.64 | 0.16 | 0.04 | 0.01 | 0.0025 | 0.000625 |
|---|---|---|---|---|---|---|---|---|
| CKS | 98% | 99% | 98% | 90% | 68% | 36% | 8% | |
| KLT | | | 98% | 89% | 81% | 57% | 24% | 5% |

**Claims**

1. A compound of the formula

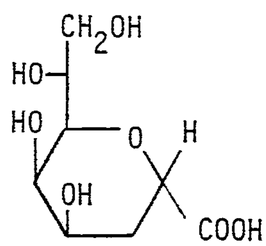

I

or a physiologically acceptable salt thereof.

2. A process for the preparation of a compound of the formula

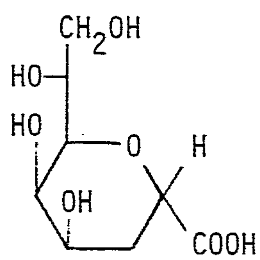

I

or a physiologically acceptable salt thereof, in which the process comprises

A) epimerization of a compound of the formula

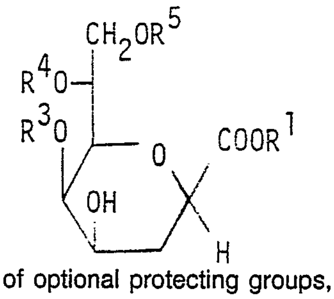

IIA

followed by removal of optional protecting groups, where in the formula $R^2$-$R^5$ are the same or different and selected from hydrogen or a protecting group stable towards base such as arylalkyl or cyclic acetal;
$R^1$ is an alkyl group;

B) cyclization of a partially protected compound of the formula

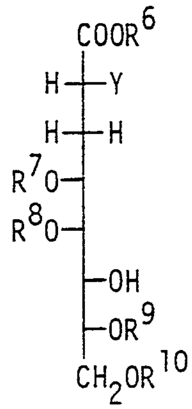

IIIA

followed by removal of optional protecting groups, where in the formula Y is a leaving group such as a halogen atom or an aryl-or alkylsulfonate group or a phosphonioxy group,
$R^7$-$R^{10}$ are the same or different and selected from hydrogen or hydroxy-protecting groups such as acyl, arylalkyl, cyclic acetal, or 2-tetrahydropyranyl;
$R^6$ is hydrogen or a carboxy-protecting group such as alkyl, arylalkyl or aryl;

C) reduction of a compound of the formula

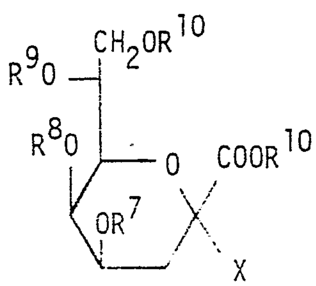

IVA or

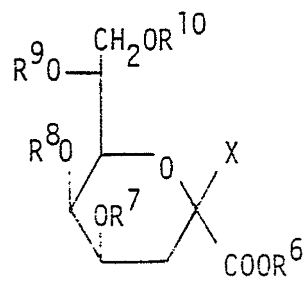

IVB

followed by removal of optional protecting groups, where in the formulas
X is a halogen atom or an alkylthio group, an arylthio group, a thiocarbonyl group or a group aryl-SCOO;
$R^6$-$R^{10}$ are protecting groups as defined under B;
whereupon a compound obtained is optionally converted into a physiologically acceptable salt.

3. A pharmaceutical preparation comprising as active ingredient a compound according to claim I or a physiologically acceptable salt thereof.

4. The use of a compound according to claim I or a physiologically acceptable salt thereof for preparation of a pharmaceutical preparation comprising as an active ingredient an amount of said compound.

5. A compound according to claim I for use as a drug.

6. A compound which after administration to a living organism is transformed into a compound

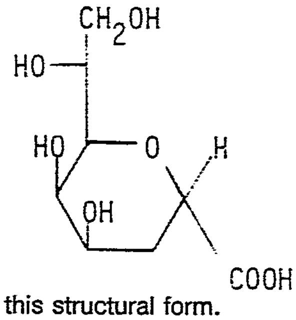

I

and exerting its effect in this structural form.

7. A chemical intermediate of the formula

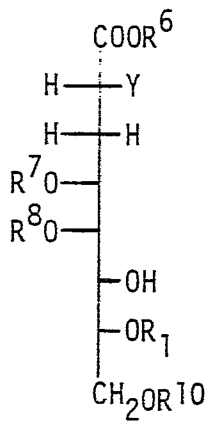

IIIA

wherein Y is a leaving group such as a halogen atom or an aryl-or alkylsulfonate group or a phosphonioxy group;

$R^7$-$R^{10}$ are the same or different and selected from hydrogen or hydroxy-protecting groups such as acyl, arylalkyl, cyclic acetal or 2-tetrahydropyranyl;

$R^6$ is hydrogen or a carboxy-protecting group such as alkyl, arylalkyl or aryl.

8. A chemical intermediate according to claim 7 wherein Y is iodo.

European Patent Office

# PARTIAL EUROPEAN SEARCH REPORT

Application number
86850439.0

| DOCUMENTS CONSIDERED TO BE RELEVANT | | | CLASSIFICATION OF THE APPLICATION (Int. Cl.4) |
|---|---|---|---|
| Category | Citation of document with indication, where appropriate, of relevant passages | Relevant to claim | |
| A | CHEMICAL COMMUNICATIONS, no. 21, 1981, pages 1139-1140, London; P.M. COLLINS et al.: "Synthesis of 3-deoxy-D-manno--octulosonic acid (KDO)." ----- | 1-5, 7-8 | |
| A | CANADIAN JOURNAL OF BIOCHEMISTRY, vol. 47, no. 7, 1969, pages 691-695; D.T. WILLIAMS et al.: "The structure, synthesis, analysis, and occurrence of 3-deoxy-D-manno-octulosonic acid." ----- | 1-5, 7-8 | |
| A | EP-A-0 160 925 (KANTO ISHI PHARMACEUTICAL CO., LTD) ----- | 1 | TECHNICAL FIELDS SEARCHED (Int. Cl.4) |
| P | US-A-4 613 589 (ABBOTT LABORATORIES) ----- | 1-5, 7-8 | |
| P | US-A-4 613 590 (ABBOTT LABORATORIES) ----- | 1-5, 7-8 | |

European Patent Office

# PARTIAL EUROPEAN SEARCH REPORT

which under Rule 45 of the European Patent Convention shall be considered, for the purposes of subsequent proceedings, as the European search report

Application number 86850439.0

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| DX | ADVANCES IN CARBOHYDRATE CHEMISTRY AND BIOCHEMISTRY, vol. 38, 1981, pages 323--388; F.M. UNGER: "The Chemistry and biological significance of 3-deoxy-D--manno-2-octulosonic acid (KDO)". * Pages 339-340; page 356 compound 56; page 376 compound 118-120; pages 365--388. * | 1-5, 7-8 | C 07 H 7/02<br>C 07 C 59/105<br>C 07 C 59/125<br>C 07 C 69/675<br>C 07 C 69/708<br>A 61 K 31/70 |
| | ----- | | |
| DX | AMERICAN CHEMICAL SOCIETY SYMPOSIUM SERIES 231, 1983, pages 141-169, Washington DC; P.H. RAY et al.: "Synthesis and use of 3-deoxy-D--manno-2-octulosonate (KDO) in Escherichia coli. Potential sites of inhibition." * Pages 141 and 154-156, "Discussion" pages 164-168. * | 1-5, 7-8 | |
| | ----- | | |
| | -/- | | |

TECHNICAL FIELDS SEARCHED (Int. Cl.4)

C 07 H
C 07 C
A 61 K

## INCOMPLETE SEARCH

The Search Division considers that the present European patent application does not comply with the provisions of the European Patent Convention to such an extent that it is not possible to carry out a meaningful search into the state of the art on the basis of some of the claims.

Claims searched completely:
Claims searched incompletely:
Claims not searched: 6
Reason for the limitation of the search:

The compounds covered by this claim are not specified. Only the compounds formed from the unspecified compounds are given.

| Place of search | Date of completion of the search | Examiner |
|---|---|---|
| STOCKHOLM | 17-03-1987 | CLAESSON G. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document